# EUROPEAN PATENT APPLICATION

(11) **EP 1 688 158 A1**
(43) Date of publication of application: **09.08.2006**
(21) Application number: 05425056.8
(22) Date of filing: 07.02.2005
(51) Int. Cl.: A61M 25/00, A61M 37/00, A61B 1/00

(54) **An endoscopic device**

(71) Applicant: Cennamo, Luigi, 17055 Toirano (Savona) (IT)
(72) Inventor: Cennamo, Luigi, 17055 Toirano (Savona) (IT)
(74) Representative: Lanzoni, Luciano

(57) **Abstract**

An endoscopic device comprises: a main core or duct (1), housing at least one operating and/or exploratory unit (2); an outer sheath (3), covering the main duct (1) and made of an elastically deformable material; and means (4) designed to deform at least one portion (5) of the sheath (3), so as to create a free passage zone (6) adjacent to the main duct (1).

## Description

The present invention relates to an endoscopic device.

At present, in the medical sector said endoscopic devices are used for internal examinations of patients (for example, tracheo - gastric explorations), or even micro-surgery on internal tissues, etc.

These devices basically consist of:
- a flexible main core or duct, with a substantially circular cross-section, and extending mainly longitudinally; the duct housing one or more channels, parallel with one another and having different diameters, occupied by corresponding accessories or operating units (for example, fibre optics, micro-cameras, micro-surgical tools, probes, etc.) which come out of the free operating end of the duct;
- an outer sheath which encompasses the main duct, protecting it, even from a hygienic viewpoint, and allowing the endoscope to slide correctly along the zones to be explored.

This endoscopic device structure has a basic disadvantage which is the fact that, due to the objective need to keep the size of the device cross-section within predetermined values (dictated by the need to insert it in small parts of the body), the maximum diameter of the channels which can be used is not sufficient or proportionate with the dimensions of particular operating units for exploration or micro-surgical operations.

This also means limiting, at the design stage, further and improved accessories for this type of inspection or operating unit.

The aim of the present invention is, therefore, to provide a solution to the above-mentioned disadvantage.

According to the invention this aim is achieved by an endoscopic device comprising: a main core or duct, housing at least one operating and/or exploratory unit; an outer sheath, covering the main duct and made of an elastically deformable material; and means designed to deform at least one portion of the sheath so as to create a free passage zone adjacent to the main duct.

The technical features of the present invention, in accordance with the above aims, are apparent in the claims herein, and the advantages are more clearly described in the detailed description below, with reference to the accompanying drawings, which illustrate a preferred embodiment of the invention, without limiting the scope of its application, and in which:
- Figure 1 is a schematic perspective view with some parts cut away to better illustrate others of an endoscopic device according to the present invention, in an insertion configuration;
- Figure 2 is a schematic perspective view with some parts cut away to better illustrate others of the device illustrated in Figure 1 in an operating configuration, that is to say, with an auxiliary channel opened up;
- Figure 3 is a schematic perspective view with some parts cut away to better illustrate others of the device illustrated in Figure 2, in an operating configuration, that is to say with an auxiliary channel opened up and occupied by an accessory or operating unit;
- Figures 4 and 5 are schematic front views with some parts cut away to better illustrate others of an alternative embodiment of the device illustrated in the previous figures, respectively in an insertion configuration and in an operating configuration.

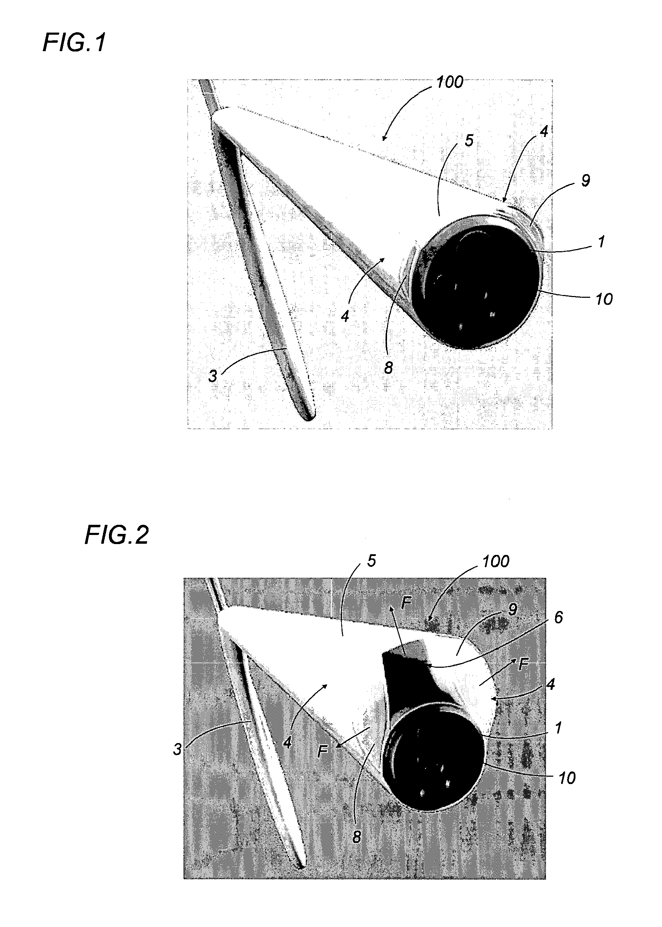

With reference to the accompanying drawings, and in particular with reference to Figure 1, the endoscopic device in question, labelled 100 as a whole, is used for internal examinations of patients (for example, tracheo - gastric explorations), or micro-surgery on internal tissues, etc.

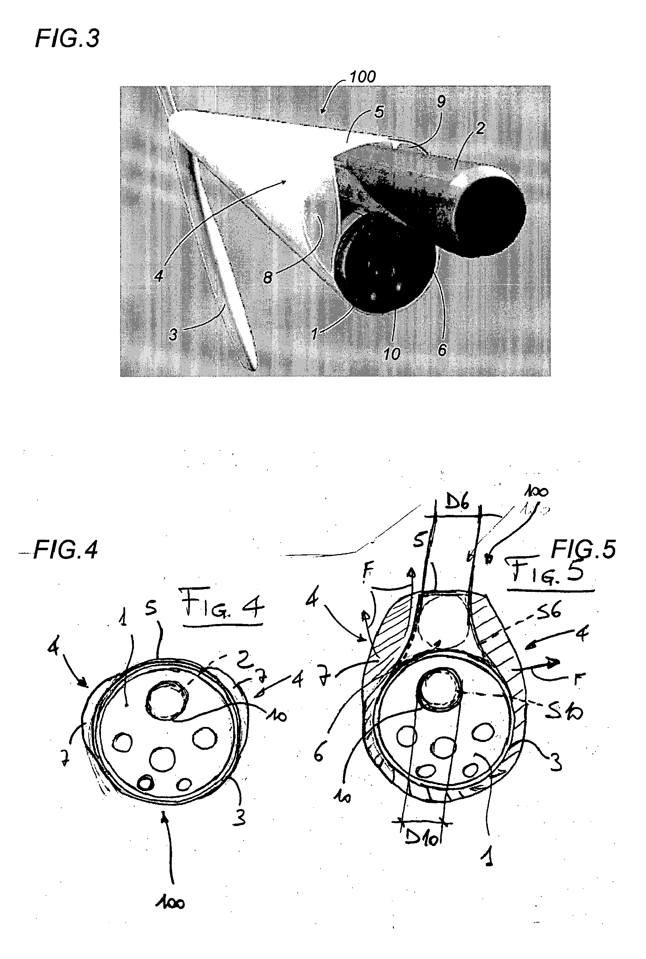

This device basically comprises:
- a main core or duct 1, housing at least one operating and/or exploratory unit 2 (illustrated with a dashed line in Figure 4 since it is of the known type and is not strictly part of the invention);
- an outer sheath 3, covering the main duct 1 and made of elastically deformable material (for example, bio-compatible rubber material);
- means 4 designed to deform at least one portion 5 of the sheath 3, so as to create a free passage zone 6 adjacent to the main duct 1.

The above-mentioned main duct 1 consists of a cylindrical duct with a circular cross-section and has at least one channel 10 for housing a unit 2.

The accompanying drawings illustrate a single channel 10, by way of example and without limiting the scope of the invention, whilst other zones are visible already set up to obtain respective channels of different sizes for housing other accessories or for other functions, not described herein.

This channel 10 has a predetermined passage area S10 (illustrated with a dashed line in Figure 5) for the unit 2 which may also be sized as a unit 2 passage working diameter D10.

In the embodiment illustrated, the above-mentioned free passage zone or auxiliary duct 6, for insertion of a unit 2, is created by the sheath 3 deformation means 4, and has a passage area S6 (illustrated with a dashed line) greater than the above-mentioned passage area S10 of the main duct 1 channel 10.

Similarly, the auxiliary duct 6 has a passage working diameter D6 greater than the passage diameter D10 of the main duct 1 channel 10.

In the construction solution illustrated in Figures 4 and 5, the deformation means 4 may comprise at least one pad 7 or chamber which can be inflated using fluid (for example air), formed between two walls or sheets of the sheath 3.

This inflatable chamber occupies a semi-circular section of the sheath 3 so as to allow, in an inflated operating configuration (Figure 5), formation of the free passage zone 6. The latter is delimited by at least three adjacent surfaces obtained from the deformed sheath 3.

As illustrated in Figures 1 to 3, as an alternative to what is specified above, the deformation means 4 may comprise a pair of pads 8 and 9 or chambers which can be inflated with fluids, independent of one another and inserted between two walls or sheets of the sheath 3. These two independent pads 8 and 9 are located on opposite sides of a hollow section or portion 5 of the sheath 3 (that it to say, made with a double sheet) so that, with the pads 8 and 9 in an inflated operating configuration (see Figure 2), the free passage zone 6 can be obtained from the hollow portion 5 of the sheath 3. The free passage zone 6 is preferably delimited by four adjacent surfaces obtained from the deformed sheath 3 using sections of its sheets.

Therefore, in practice the endoscopic device described above may be inserted in the exploration or operating zone in a standard configuration (that is to say, with the pads deflated) and with a standard diameter.

Then, when it reaches the selected target, the pad or pads are inflated to create the auxiliary duct (see Figures 2 and 5 and the arrows F) and allow the passage of exploratory units (fibre optics, television cameras, probes, etc.) or operating units (micro-surgery instruments) to support or as a alternative to those already present on the main duct.

At the end of the procedure, the units are withdrawn and the pads deflated to allow the device to be taken out.

The endoscopic device structured in this way, therefore, achieves the preset aims with a simple double-sheet sheath, made of deformable rubber material.

This sheath comprises one or two air chambers designed to create an auxiliary operating channel when the chambers are inflated.

This possibility brings advantages such as:
- the possibility of using more operating units simultaneously and with dimensions greater than the current standards, the auxiliary duct being bigger than the largest channel present on the main duct;
- standard dimensions during insertion in and removal from the selected zones;
- the possibility of designing larger instruments with greater operating capacity than the current standard instruments.

The invention described may be subject to modifications and variations without thereby departing for the scope of the inventive concept. Moreover, all details of the invention may be substituted by technically equivalent elements.

## Claims

1. An endoscopic device **characterised in that** it comprises:
- a main core or duct (1), housing at least one operating and/or exploratory unit (2);
- an outer sheath (3), covering the main duct (1) and made of an elastically deformable material;
- means (4) designed to deform at least one portion (5) of the sheath (3) to create a free passage zone (6) adjacent to the main duct (1).

2. The device according to claim 1, in which the main duct (1) has at least one channel (10) for housing the unit (2), having a predetermined passage area (S10), **characterised in that** the auxiliary free passage zone or duct (6) has a passage area (S6) greater than the passage area (S10) of the main duct (1) channel (10), of which there is at least one.

3. The device according to claim 1, in which the main duct (1) has at least one channel (10) for housing the unit (2), having a predetermined passage working diameter (D10), **characterised in that** the auxiliary free passage zone or duct (6) has a passage working diameter (D6) greater than the passage working diameter (D10) of said main duct (1) channel (10), of which there is at least one.

4. The device according to claim 1, **characterised in that** the deformation means (4) comprise at least one pad (7) or chamber inflatable with fluid, formed between two walls of the sheath (3) and occupying a semi-circular section of the sheath (3) so that, in an inflated operating configuration, said free passage zone (6), delimited by at least three adjacent surfaces, can be obtained from the deformed sheath (3).

5. The device according to claim 1, **characterised in that** the deformation means (4) comprise a pair of pads (8, 9) or chambers inflatable with fluids, formed between two walls of the sheath (3) and positioned on opposite sides of a hollow section or portion (5) of the sheath (3) so as to allow the formation, with the pads (8, 9) in an inflated operating configuration, of the free passage zone (6), in said hollow portion (5) of the sheath (3).

6. The device according to claim 4 or 5, **characterised in that** the free passage zone (6) is delimited by four adjacent surfaces obtained from the deformed sheath (3).
